# EUROPEAN PATENT SPECIFICATION

(11) **EP 2 828 275 B1**
(45) Date of publication and mention of the grant of the patent: **01.03.2017**
(21) Application number: 13764216.1
(22) Date of filing: 20.03.2013
(51) Int. Cl.: C07H 15/18, C07H 3/06, C07H 17/04

(54) **SYNTHESIS OF THE TRISACCHARIDE 3-O-FUCOSYLLACTOSE AND INTERMEDIATES THEREOF**
SYNTHESE VON TRISACCHARID 3-O-FUCOSYLLACTOSE UND ZWISCHENPRODUKTE DAVON
SYNTHÈSE DE TRISACCHARIDE DE TYPE 3-O-FUCOSYLLACTOSE ET DE SES INTERMÉDIAIRES

(30) Priority: 20.03.2012 DK 201270124
(43) Date of publication of application: 28.01.2015
(73) Proprietor: Glycom A/S, 2970 Hørsholm (DK)
(72) Inventor: RÖHRIG, Christoph, 78357 Mühlingen (DE); DEKANY, Gyula, Sinnamon Park Queensland 4073 (AU); MATWIEJUK, Martin, 22115 Hamburg (DE); DEMKÓ, Sándor, 4032 Debrecen (HU)
(74) Representative: Inspicos P/S
(86) International application number: PCT/DK2013/050078
(87) International publication number: WO 2013/139344

(56) References cited:
- WO-A1-2012/113405
- WO-A1-2012/127410
- WO-A1-2014/075680
- FERNANDEZ-MAYORALAS A.: 'Synthesis of 3- and 2'- fucosyl-lactose and 3,2'-difucosyl-lactose from partially benzylated lactose derivatives' CARBOHYDRATE RESEARCH vol. 154, no. 1, 15 October 1986, pages 93 - 101, XP026618813
- PEREIRA C. L.: 'Synthesis of human milk oligosaccharides:2'- and 3'-fucosyllactose' HETEROCYCLES vol. 84, no. 1, 2012, XP008174628

## Description

### FIELD OF THE INVENTION

The present invention relates to the synthesis of the trisaccharide 3-*O*-fucosyllactose, intermediates used in the synthesis and the synthesis of the intermediates.

### BACKGROUND OF THE INVENTION

In recent years, efforts have increasingly been made to produce industrially complex carbohydrates, such as secreted oligosaccharides. This has been due to the roles of such compounds in numerous biological processes in living organisms. Secreted oligosaccharides, such as human milk oligosaccharides ("HMOs"), have become particularly important commercial targets for nutrition and therapeutic applications. However, the synthesis and purification of these oligosaccharides have remained a challenging task. One of the simplest important human milk oligosaccharides is 3-*O*-fucosyllactose (β-D-galactopyranosyl-(1→4)-(α-L-fucopyranosyl-(1→3))-D-glucose, "3-FL"):

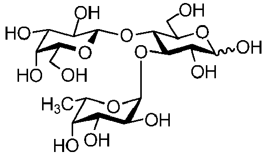

Several biological activities of 3-FL have been reported including its prebiotic, antibacterial, antiviral, immune system-enhancing and brain development-enhancing activities. These activities of 3-FL have made it a potentially attractive additive for nutritional and therapeutic products.

With an avarage concentration of about 0.7 g/l, 3-FL belongs to the 5 most abundant HMOs in mother's milk. But due to the complexity of the HMO fraction its isolation from natural sources has required lengthy and complicated chromatographic procedures, making its isolation very costly.

Although 3-FL has also been synthesized by enzymatic, biotechnological and chemical processes, no commercially attractive process has yet been developed. Bacterial fermentation in the presence of lactose of metabolically engineered *E. coli* having an *H. pylori* α-1-3-fucosyltransferase gene has produced predominantly fucosylated higher oligosaccharides and only minor amounts of 3-FL (Dumon et al. Biotechnol. Prog. 20, 412 [2004]). Recently this technology has been developed to produce 3-FL in 0.02-0.05 g/l concentration (WO 2010/142305).

Reported chemical methods either have not provided crystalline precursors which could be easily purified (Fernandez-Mayoralas et al. Carbohydr. Res. 154, 93 [1986]) or have required many difficult and costly steps to remove protecting groups from precursors and then purify the final product which have made such methods too costly for commercialization (Pereira et al. Heterocycles 84, 637 [2012]).

### SUMMARY OF THE INVENTION

The present invention provides a commercially attractive process for making 3-FL in high yield and purity.

The first aspect of the present invention relates to a compound of formula **1** 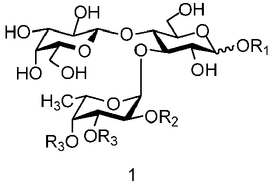 wherein R₁ and R₂ are independently a group removable by hydrogenolysis, and R₃ is selected from a group removable by hydrogenolysis and H,
or a hydrate or solvate thereof. Preferably in the compound of formula **1** R₁ is selected from benzyl, 4-methylbenzyl, naphthylmethyl, 4-phenylbenzyl, 4-chlorobenzyl, 4-methoxybenzyl, 3,4-dimethoxybenzyl, 2,4,6-trimethylbenzyl and 2,3,4,5,6-pentamethylbenzyl, more preferably from benzyl and 4-methylbenzyl; R₂ is selected from benzyl, 4-methylbenzyl, naphthylmethyl, benzyloxycarbonyl, 4-phenylbenzyl, 4-chlorobenzyl, 4-methoxybenzyl, 3,4-dimethoxybenzyl, 2,4,6-trimethylbenzyl and 2,3,4,5,6-pentamethylbenzyl, more preferably from benzyl and 4-methylbenzyl; R₃ is selected from benzyl, 4-methylbenzyl, naphthylmethyl, benzyloxycarbonyl, 4-phenylbenzyl, 4-chlorobenzyl, 4-methoxybenzyl, 3,4-dimethoxybenzyl, 2,4,6-trimethylbenzyl, 2,3,4,5,6-pentamethylbenzyl and H, more preferably from benzyl, 4-methylbenzyl and H; and -OR₁ is in β-orientation.

The second aspect of this invention relates to a method for making 3-FL comprises the step of subjecting a compound of formula **1** or a hydrate or solvate thereof to hydrogenolysis. In this regard, the hydrogenolysis is preferably carried out in water, in one or more C₁-C₆ alcohols or in a mixture of water and one or more C₁-C₆ alcohols in the presence of a palladium or a Raney nickel catalyst, more preferably in the presence of palladium on charcoal or palladium black. It is also preferred in carrying out this method that, in the compound of formula **1** R₁ is selected from benzyl, 4-methylbenzyl, naphthylmethyl, 4-phenylbenzyl, 4-chlorobenzyl, 4-methoxybenzyl, 3,4-dimethoxybenzyl, 2,4,6-trimethylbenzyl and 2,3,4,5,6-pentamethylbenzyl, more preferably from benzyl and 4-methylbenzyl; R₂ is selected from benzyl, 4-methylbenzyl, naphthylmethyl, benzyloxycarbonyl, 4-phenylbenzyl, 4-chlorobenzyl, 4-methoxybenzyl, 3,4-dimethoxybenzyl, 2,4,6-trimethylbenzyl and 2,3,4,5,6-pentamethylbenzyl, more preferably from benzyl and 4-methylbenzyl; R₃ is selected from benzyl, 4-methylbenzyl, naphthylmethyl, benzyloxycarbonyl, 4-phenylbenzyl, 4-chlorobenzyl, 4-methoxybenzyl, 3,4-dimethoxybenzyl, 2,4,6-trimethylbenzyl, 2,3,4,5,6-pentamethylbenzyl and H, more preferably from benzyl, 4-methylbenzyl and H; and -OR₁ is in β-orientation.

The third aspect of this invention relates to a method for making a compound of formula **1** from a compound of formula **2** 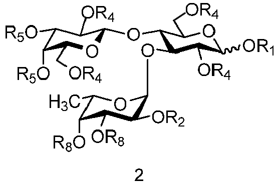 wherein R₁ and R₂ each are as defined above; R₄ is acyl; R₅ is acyl, or two R₅ groups together form a moiety 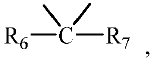 wherein R₆ and R₇ are independently selected from alkyl and phenyl, or wherein R₆ and R₇ together with the carbon atom to which they are attached form a cycloalkylidene; and R₈ is selected from a group removable by hydrogenolysis and acyl, or two R₈ groups together form a moiety 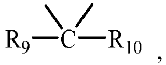 wherein R₉ and R₁₀ independently are selected from alkyl and phenyl, or wherein R₉ and R₁₀ together with the carbon atom
to which they are attached form a cycloalkylidene;
characterized by the steps of converting the compound of formula **2** into a compound of formula **1** or a hydrate or solvate thereof by:
a) deacylating the R₄ acyl groups and any R₅ and R₈ acyl groups of the compound of formula **2;** and optionally
b) removing any moiety 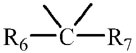 and any moiety 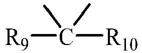 by treatment with an acid.

The fourth aspect of this invention relates to a method of making 3-FL characterized by the steps of making a compound of formula **1** or a hydrate or solvate thereof from a compound of formula **2** by the method of the third aspect of this invention and then subjecting the compound of formula **1** or a hydrate or solvate thereof to hydrogenolysis by the method of the second aspect of the invention.

The fifth aspect of this invention relates to a compound of formula **2A** 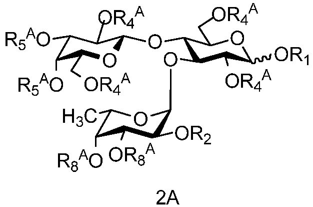 wherein R₁ and R₂ are independently a group removable by hydrogenolysis; R₄^{A} is selected from acyl and H; R₅^{A} is selected from acyl and H, or two R₅^{A} groups together form a moiety 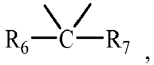 wherein R₆ and R₇ are independently selected from alkyl and phenyl, or wherein R₆ and R₇ together with the carbon atom to which they are attached form a cycloalkylidene; and R₈^{A} is selected from acyl and H, or two R₈^{A} groups together form a moiety 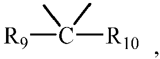 wherein R₉ and R₁₀ independently are selected from alkyl or phenyl, or wherein R₉ and R₁₀ together with the carbon atom to which they are attached form a cycloalkylidene, provided that R₄^{A}, R₅^{A} and R₈^{A} are not all H; or a hydrate or solvate thereof.

### DETAILED DISCLOSURE OF THE INVENTION

In this invention, the term "alkyl" means a linear or branched chain saturated hydrocarbon group with 1-6 carbon atoms, such as methyl, ethyl, *n*-propyl, *i*-propyl, *n*-butyl, *i*-butyl, *s*-butyl, *t*-butyl, *n*-hexyl, etc. The term "cycloalkylidene" means a bivalent cyclic hydrocarbon ring or group having 3-8 carbon atoms, such as cyclopropylidene, cyclopentylidene, cyclohexylidene, cycloheptylidene, etc. The term "aryl" means a homoaromatic group such as phenyl or naphthyl. The term "acyl" means a Q-C(=O)-group, wherein Q may be H, alkyl (see above) or aryl (see above), such as formyl, acetyl, propionyl, butyryl, pivaloyl, benzoyl, etc. The term "benzyl" means a phenylmethyl group. The term "alkyloxy" or "alkoxy" means an alkyl group attached to a parent molecular moiety through an oxygen atom, such as methoxy, ethoxy, *t*-butoxy, etc.

Herein, the above-mentioned alkyl (also as a part of acyl), cycloalkylidene or aryl (also as a part of acyl) groups can either be unsubstituted or substituted one or several times, preferably 1-5 times, more preferably 1-3 times. The substituents can be alkyl (for aryl and cycloalkylidene), hydroxy, alkoxy, carboxy, oxo (forming a keto or aldehyde function), alkoxycarbonyl, alkylcarbonyl, formyl, aryl, aryloxycarbonyl, aryloxy, arylamino, arylcarbonyl, amino, mono- and dialkylamino, carbamoyl, mono- and dialkyl-aminocarbonyl, alkylcarbonylamino, cyano, alkanoyloxy, nitro, alkylthio and/or halogen (F, Cl, Br, I).

Also herein, the term "group removable by hydrogenolysis" means a protecting group that has a C-O bond with the oxygen of the -OR₁,-OR₂ -OR₃, -OR₈, -OR₈^{B}, and -OR₈^{C} groups of the compounds of formulas **1, 2, 2B** and **2C,** and that can be cleaved by hydrogen in the presence of a catalytic amount of palladium, Raney nickel or any other conventional hydrogenolysis catalyst to regenerate the OH group. Such protecting groups are described in Wuts and Greene: Protective Groups in Organic Synthesis, John Wiley & Sons, 2007**,** and include benzyl, diphenylmethyl (benzhydryl), 1-naphthylmethyl, 2-naphthylmethyl, triphenylmethyl (trityl) and benzyloxycarbonyl groups, each of which can be optionally substituted by one or more of the following groups: alkyl, alkoxy, phenyl, amino, acylamino, alkylamino, dialkylamino, nitro, carboxyl, alkoxycarbonyl, carbamoyl, *N*-alkylcarbamoyl, *N,N*-dialkylcarbamoyl, azido, halogenalkyl or halogen. Preferably, such substitution, if present, is on the aromatic ring(s). A preferred protecting group is benzyl or naphthylmethyl optionally substituted with one or more of the following groups: phenyl, alkyl, alkoxy and halogen, more preferably benzyl, 4-methylbenzyl, naphthylmethyl, 4-phenylbenzyl, 4-chlorobenzyl, 4-methoxybenzyl, 3,4-dimethoxybenzyl, 2,4,6-trimethylbenzyl and 2,3,4,5,6-pentamethylbenzyl, particularly unsubstituted benzyl, 4-chlorobenzyl, 3-phenylbenzyl and 4-methylbenzyl groups. These preferred and particularly preferred protecting groups have the advantage that the by-products of their hydrogenolysis are exclusively toluene or substituted toluene. Such by-products can easily be removed, from water-soluble oligosaccharide products via evaporation and/or extraction processes.

In accordance with the first aspect of this invention, compounds of the following formula **1** or a hydrate or solvate thereof are provided 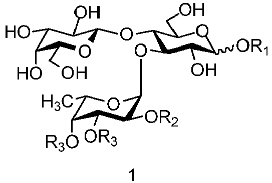 wherein R₁ and R₂ are independently a group removable by hydrogenolysis; and R₃ is selected from a group removable by hydrogenolysis and H.

These compound can be isolated as α or β anomers or anomeric mixtures of α and β isomers. They can be isolated in pure form as crystalline solids, but can also be oils, syrups, precipitated amorphous solids or spray dried solids. If crystalline, these compounds can exist either in an anhydrous or a hydrated crystalline form, incorporating one or more molecules of water into their crystal structures. Similarly, these can exist as crystalline substances incorporating ligands such as organic molecules and/or ions into their crystal structures.

Preferably compounds of formula **1** are crystalline materials. Crystalline partially benzylated 3-FL precursors are valuable and highly advantageous final process intermediates for use in making 3-FL of high purity, especially in a large or industrial scale. Generally, crystallization and/or recrystallization are the simplest and cheapest methods to isolate a product or its precursor from a reaction mixture, separate it from contaminants and obtain it in pure form. Isolation or purification that uses crystallization makes any technological process more efficient. Because R₁, R₂ and optionally R₃ in the compounds of formula **1** are benzyl/substituted benzyl protecting groups, their removal from the compounds can occur nearly quantitatively without significant by-product formation even under gentle hydrogenolysis conditions. These protecting groups are converted exclusively into toluene/substituted toluene during hydrogenolysis, and they can easily be removed from the water soluble 3-FL via conventional evaporation and/or extraction processes. Thus the chemical purity of the 3-FL that can be obtained is comparable to that of the compound of formula **1,** from which the 3-FL was formed. As 3-FL is not produced as a crystalline material, costly and cumbersome techniques would be needed to purify it except for the fact that its precursors of formula **1** can be obtained in crystalline, and therefore highly pure, form.

In preferred compounds of formula **1,** R₁ is selected from benzyl, 4-methylbenzyl, naphthylmethyl, 4-phenylbenzyl, 4-chlorobenzyl, 4-methoxybenzyl, 3,4-dimethoxybenzyl, 2,4,6-trimethylbenzyl and 2,3,4,5,6-pentamethylbenzyl, more preferably from benzyl and 4-methylbenzyl; R₂ is selected from benzyl, 4-methylbenzyl, naphthylmethyl, benzyloxycarbonyl, 4-phenylbenzyl, 4-chlorobenzyl, 4-methoxybenzyl, 3,4-dimethoxybenzyl, 2,4,6-trimethylbenzyl and 2,3,4,5,6-pentamethylbenzyl, preferably from benzyl and 4-methylbenzyl; R₃ is selected from benzyl, 4-methylbenzyl, naphthylmethyl, benzyloxycarbonyl, 4-phenylbenzyl, 4-chlorobenzyl, 4-methoxybenzyl, 3,4-dimethoxybenzyl, 2,4,6-trimethylbenzyl, 2,3,4,5,6-pentamethylbenzyl and H, preferably from benzyl, 4-methylbenzyl and H; and -OR₁ is in β-orientation. Especially preferred compounds of formula **1** are of the following formulae **1A** and **1B**:

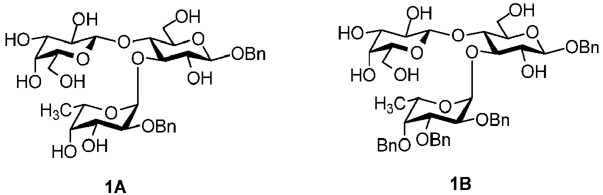

Compounds of formula **1** can be used for the preparation of 3-FL itself and 3-FL derivatives using conventional chemical/enzymatic methodologies. The compounds of formula **1** can also be used as precursors for making numerous other human milk oligosaccharides. The compounds of formula **1** can also be used as precursors for making other complex oligosaccharides and glycoconjugates suitable for therapeutic and/or nutritional use.

In accordance with the second aspect of this invention, a method is provided for synthesizing 3-FL by hydrogenolysis of a compound of formula **1.** This hydrogenolysis can be carried out in a conventional manner. Preferably, the hydrogenation is carried out by treating the compound of formula **1** with hydrogen in the presence of a catalyst in a protic solvent or in a mixture of protic solvents. The protic solvent can be water, acetic acid or a C₁-C₆ alcohol. A mixture of one or more protic solvents with one or more aprotic organic solvents that are partially or fully miscible with the protic solvent(s), such as THF, dioxane, ethyl acetate or acetone can be used. Water, one or more C₁-C₆ alcohols, or a mixture of water and one or more C₁-C₆ alcohols is preferably used as the solvent system. Solutions and suspension containing a compound of formula **1** in any concentrations with the above-mentioned solvent(s) can also be used. The reaction mixture can be stirred at 10-100 °C, preferably at 20-50 °C, more preferably 35-40 °C in hydrogen gas atmosphere of 1-50 bar, preferably 5-20 bar in the presence of a catalyst such as palladium or Raney nickel, preferably palladium on charcoal or palladium black. A catalyst concentration of 0.1-10 %, preferably 0.15-5 %, more preferably 0.25-2.25 %, based on the weight of the compound of formula **1** can be used. Alternatively, transfer hydrogenolysis can be carried out. In this regard, hydrogen can be generated *in situ* from cyclohexene, cyclohexadiene, formic acid or ammonium formate. The pH of the hydrogenolysis mixture is preferably neutral, but organic or inorganic bases/acids and/or basic and/or acidic ion exchange resins can also be used to improve the kinetics of the hydrogenolysis. The use of basic substances is especially preferred when halogen substituent(s) are present on the substituted benzyl groups of the compound of formula **1.** Preferred organic bases include triethylamine, diisopropyl ethylamine, ammonia, ammonium carbamate and diethylamine. An acid can be advantageously used as a co-solvent or additive when multiple benzyl groups have to be removed from the compound of formula **1.** Preferred acids include formic acid, acetic acid, propionic acid, chloroacetic acid, dichloroacetic acid, trifluoroacetic acid, HCl and HBr.

By this method, 3-*O*-fucosyllactose can be readily produced in high yield and purity, even in industrial quantities. In this regard, the 3-FL so produced can be isolated as an amorphous solid by precipitation from water or an organic solvent or an aqueous solution or - after filtration of the catalyst - from the solution in which it was formed from the compound of formula **1.** This can be done simply by cooling, or adding an ether such as MTBE, diethyl or diisopropyl ether, a C₁-C₆ alcohol, acetone or a mixture thereof to the solution. Alternatively 3-FL can also be isolated by freeze drying and spray drying.

In one preferred embodiment, after the hydrogenolysis of a compound of formula **1,** the reaction mixture is filtered, preferably concentrated by removing any organic solvent and then subjected to precipitation, spray drying or freeze drying to produce an anhydrous or a hydrated 3-FL (water content 0-20 %).

In another preferred embodiment, after the hydrogenolysis of a compound of formula **1,** the reaction mixture is filtered and then preferably concentrated to produce a 3-FL aqueous solution or syrup with a 3-FL concentration of 10-95 %.

The 3-FL produced by the hydrogenolysis of a compound of formula **1** can be isolated as an amorphous, freeze dried or spray dried solid or as aqueous liquid or syrup. The 3-FL can be isolated with high purity suitable for infant nutritional use (e.g., for use in infant formulas, infant cereals, clinical infant nutritional products etc.) Indeed, both solid and liquid forms of the 3-FL produced by this invention are suitable for general nutritional use by infants, toddlers, children, adults and the elderly. This 3-FL can also be used as a food additive, dietary supplement, and a component of alcoholic and non-alcoholic beverages (e.g., soft drinks, fruit juices, bottled water, wine and beer) or as a therapeutic agent (e.g., to prevent bacterial and viral infections, to avoid diarrhoea and to enhance human immune systems and brain development). This 3-FL can also be used in veterinary applications (e.g., to fight infectious diseases of domesticated animals). This 3-FL can also be used as a monomer for preparing polymeric/polymer-mounted products, providing multivalent binding for bacteria and viruses and for preparing other HMOs by chemical and/or enzymatic processes (e.g., by further fucosylation, sialylation and/or extension of the core 3-FL structure via *N*-acetyl lactosaminylation/*N*-acetyl isolactosamination).

In accordance with the third aspect of this invention, a method is provided for making a compound of formula **1** from a compound of formula **2** 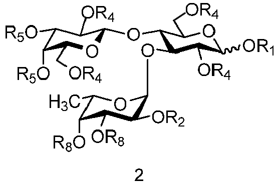 wherein R₁ and R₂ each are as defined above; R₄ is acyl; R₅ is acyl, or two R₅ groups together form a moiety 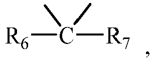 wherein R₆ and R₇ are independently selected from alkyl and phenyl, or wherein R₆ and R₇ together with the carbon atom to which they are attached form a cycloalkylidene; and R₈ is selected from a group removable by hydrogenolysis and acyl, or two R₈ groups together form a moiety 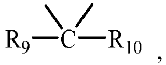 wherein R₉ and R₁₀ independently are selected from alkyl and phenyl, or wherein R₉ and R₁₀ together with the carbon atom
to which they are attached form a cycloalkylidene,
or a hydrate or solvate thereof comprising the steps of:
a) deacylating the R₄ acyl groups and any R₅ and R₈ acyl groups of the compound of formula **2;** and optionally
b) removing any moiety 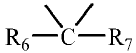 and any moiety 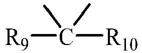 from the compound of formula **2** by treatment with an acid.

Deacylation of the R₄ acyl groups and any R₅ and R₈ acyl groups can be carried out in a conventional manner to remove acyl groups. Acyl groups can be removed in a base catalysed transesterification deprotection reaction, so any acyl protecting groups for hydroxyls are removed in an alcohol solvent such as methanol, ethanol, propanol or *t*-butanol in the presence of an alcoholate such as NaOMe, NaOEt or KO^{t}Bu at 20-100 °C. The alcohol and the alcoholate should be matched. The use of a co-solvent as toluene or xylene can be beneficial to control particle size of the product and to avoid gel formation. Preferably, a catalytic amount of NaOMe is used in methanol (Zemplén de-*O*-acylation). Acyl groups can also be removed by a base catalysed hydrolysis in water, an alcohol or a water-organic solvent mixture in homogeneous or heterogeneous reaction conditions at 0-100 °C. Preferably, a strong base is used such as LiOH, NaOH, KOH, Ba(OH)₂, K₂CO₃, a basic ion exchange resin or a tetraalkylammonium hydroxide. In a preferred embodiment, the base is NaOH and the solvent is methanol. By aminolysis, i.e. *N*-acyl transfer based deprotection, acyl groups can also be removed with ammonia, hydrazine, substituted hydrazine, ethylene diamine or primary amines in water, alcohol or water-organic solvent mixtures at 20-120 °C.

Any 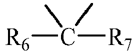 and/or 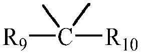 protecting moieties can be removed by treatment with an acid in a conventional manner. By treatment with water acidified to pH>1-2, any such protecting cyclic acetal and/or ketal moieties can be removed simultaneously or successively to regenerate the 1,2-diol(s). Although, the compound of formula **2** also has acyl protecting groups which can also be removed by strong acidic hydrolysis (pH<1-2) and interglycosidic linkages that can also be split by strong acidic hydrolysis (pH<1-2), one skilled in the art can readily select reaction conditions for removing the protecting cyclic acetal or ketal moieties while leaving intact acyl protecting groups and interglycosidic linkages. Water, which serves as a reagent for removing the protecting cyclic acetal or ketal moieties, can also serve as a solvent or co-solvent in this hydrolysis reaction. In this reaction, organic protic or aprotic solvents which are stable under acidic conditions and miscible fully or partially with water, such as C₁-C₆ alcohols, acetone, THF, dioxane, ethyl acetate or MeCN, can be used in a mixture with water, and with protic acids such as acetic acid, trifluoroacetic acid, HCl, formic acid, sulphuric acid, perchloric acid, oxalic acid, *p-*toluenesulfonic acid, benzenesulfonic acid or a cation exchange resin in from catalytic amounts to large excesses. The hydrolysis can be carried out at temperatures of 20 °C to reflux until reaching completion which can take about 2 hours to 3 days depending on temperature, concentration and pH. Preferred are: an aqueous solution of an organic acid such as acetic acid, formic acid, chloroacetic acid or perchloric acid used at 20-75 °C; and a C₁-C₆ alcohol-water-DCM mixture in the presence of HCl, TFA or a sulfonic acid such as *p-*toluenesulfonic acid or champhorsulfonic acid. Alternatively, an anhydrous C₁-C₆ alcohol can be used for the cleavage of the acyclic/cyclic acetal/ketal moieties by a trans-acetalization/trans-ketalization process catalysed by an acid such as hydrogen chloride, sulphuric acid, perchloric acid, *p*-toluenesulfonic acid, acetic acid, oxalic acid, champhorsulfonic acid or a strong acidic ion-exchange at 20 °C to reflux. Preferably, such an acid catalysed mild hydrolysis is carried out in a mixture of water and a C₁-C₆ alcohol, preferably isopropanol, in the presence of a sulfonic acid, preferably *p*-toluenesulfonic acid.

Steps a) and b) in the method of the third aspect of this invention can be carried out in any order. Thus, deacylation of a compound of formula **2,** wherein R₄, R₅ and R₆ are independently acyls, leads directly to compounds of formula **1,** whereas deacylation of compounds of formula **2,** wherein at least one of the 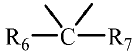 and 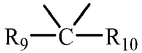 moieties is present, results in the formation of a compound of formula **2B** 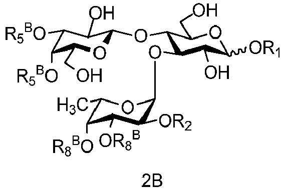 wherein R₁ and R₂ each are as defined above; R₅ is H, or two R₅^{B} groups together form a moiety 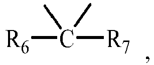 wherein R₆ and R₇ are independently selected from alkyl and phenyl, or wherein R₆ and R₇ together with the carbon atom to which they are attached form a cycloalkylidene; and R₈^{B} is selected from a group removable by hydrogenolysis and H, or two R₈^{B} groups together form a moiety 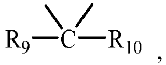 wherein R₉ and R₁₀ independently are selected from alkyl and phenyl, or wherein R₉ and R₁₀ together with the carbon atom to which they are attached form a cycloalkylidene, provided that at least one 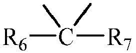 or 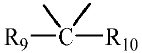 moiety is present.

The compound of formula **2B** can then be easily converted by acid treatment into a compound of formula **1.**

In a reverse order of deprotection, a compound of formula **2,** wherein at least one 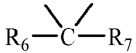 or 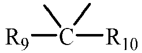 moiety is present, can be subjected to acid treatment to obtain a compound of formula **2C** 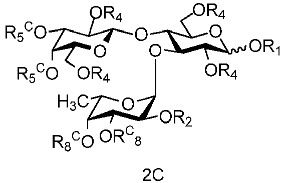 wherein R₁ and R₂ each are as defined above; R₄ is acyl; R₅ is selected from H and acyl; and R₈^{C} is selected from a group removable by hydrogenolysis, acyl and H, provided that at least one of R₅^{C} and
R₈^{C} is H.

The compound of formula **2C** can then be easily converted by deacylation into a compound of formula **1.**

A compound of formula **2** (which is a fully-protected 3-FL derivative) can be synthesized via glycosylation. Thus, a glycosyl donor of formula **3** 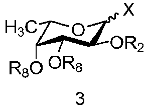 wherein R₂ and R₈ each are as defined above; and X is selected from a halogen, -OC(=NH)CCl₃, -*O*-pentenyl, -OAc, -OBz and -SR₁₁, in which R₁₁ is selected from alkyl and optionally substituted phenyl;
can be coupled to a glycosyl acceptor of formula **4** 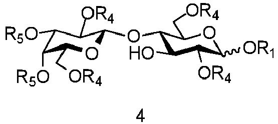 wherein R₁, R₄ and R₅ each are as defined above.

This glycosylation reaction to produce the compound of formula **2** can be carried out in a conventional manner in an aprotic solvent or in a mixture of aprotic solvents in the presence of an activator. See Demchenko (Ed.): Handbook of Chemical Glycosylation Wiley (2008). The glycosylation reaction is generally promoted by heavy metal ions, mainly mercury or silver, and Lewis acids such as trimethylsilyl triflate or BF₃-etherate.

Preferably, a glycosyl halide (i.e., X is F, Cl, Br or I) is used because of its easy accessibility and satisfactory reactivity. Typically, anomeric halides follow the reactivity order F<Cl<Br<I for nucleophilic displacement. Glycosyl fluorides can be prepared by treating the appropriate precursors such as hemiacetals, glycosyl halides, glycosyl esters and *S*-glycosides with fluorinating reagents such as HF, AgF, AgBF₄, tetrabutyl ammonium fluoride, diethylaminosulfur trifluoride, 2-fluoro-1-methylpyridinium tosylate, Selectfluor, Deoxo-Fluor or 4-methyl(difluoroiodo)-benzene.

A glycosyl trichloroacetimidate (i.e., X is -OC(=NH)CCl₃) can be prepared by adding a sugar with a free anomeric OH to trichloroacetonitrile under inorganic or organic base catalysis. The resulting glycosyl donor can be activated by a catalytic amount of a Lewis acid, such as trimethylsilyl triflate or BF₃-etherate, for the glycosylation reaction.

Glycosyl acetates or benzoates (i.e., X is -OAc or -OBz) are preferably first subjected to electrophilic activation to provide a reactive intermediate and then treated with a nucleophilic OH-acceptor. Typical activators of choice are Bronsted acids (e.g., *p*-TsOH, HClO₄ or sulfamic acid), Lewis acids (e.g., ZnCl₂, SnCl₄, triflate salts, BF₃-etherate, trityl perchlorate, AlCl₃ or triflic anhydride) or a mixture thereof.

Pentenyl glycosides (i.e. X is -*O*-(CH₂)₃-CH=CH₂) can be transglycosylated with appropriate glycosyl acceptors in the presence of a promoter such as NBS and NIS. Protic or Lewis acids (triflic acid, Ag-triflate, etc.) can enhance the reaction. The pentenyl glycosides can be prepared with the aid of *n*-pentenol by standard Fischer glycosylation of hemiacetals under acidic condition, by silver(I) salt promoted coupling of glycosyl bromides (Koenigs-Knorr method), or by glycosylation of 1-acetyl glycosides in the presence of tin(IV) chloride.

Thioglycosides (i.e., X is alkylthio- or optionally substituted phenylthio-group) can be activated by thiofilic promoters such as mercury(II) salts, Br₂, I₂, NBS, NIS, triflic acid, triflate salts, BF₃-etherate, trimethylsilyl triflate, dimethyl-methylthio sulphonium triflate, phenylselenyl triflate, iodonium dicollidine perchlorate, tetrabutylammonium iodide or mixtures thereof, preferably by Br₂, NBS, NIS or triflic acid.

Aprotic solvents such as toluene, THF, DCM, chloroform, dioxane, acetonitrile, chlorobenzene, ethylene dichloride, DMSO, DMF or *N*-methylpyrrolidone or mixtures thereof, preferably DMF, toluene, DCM or mixtures thereof, more preferably toluene or DMF-DCM mixture can be used in this glycosylation reaction at -20 to 20 °C, preferably at -10 to 5 °C, with reaction time of 5 min to 2 hours. For thiophilic activation, Br₂, NBS or NIS can be used, optionally in the presence of triflic acid or a triflate derivative. Usually a slight excess of donor (1.1-1.2 eq.) is used compared to the acceptor. For quenching the reaction, water or a C₁-C₆ alcohol is generally used, preferably an aqueous or alcoholic solution of a base like sodium carbonate, sodium bicarbonate, ammonia or triethyl amine, more preferably an aqueous Na₂S₂O₃/NaHCO₃ solution.

Preferably, the glycosyl donor is a compound of formula **3A** 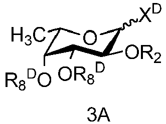 wherein R₂ is as defined above; R₈^{D} is selected from acyl and a group removable by hydrogenolysis; and X^{D} is phenylthio optionally substituted with one or more alkyl.

More preferably R₂ is selected from benzyl, 4-methylbenzyl, naphthylmethyl, 4-phenylbenzyl, 4-chlorobenzyl, 4-methoxybenzyl, 3,4-dimethoxybenzyl, 2,4,6-trimethylbenzyl and 2,3,4,5,6-pentamethylbenzyl; R₈^{D} is selected from benzyl, 4-methylbenzyl, naphthylmethyl, 4-phenylbenzyl, 4-chlorobenzyl, 4-methoxybenzyl, 3,4-dimethoxybenzyl, 2,4,6-trimethylbenzyl, 2,3,4,5,6-pentamethylbenzyl and benzoyl optionally substituted by one or more halogens; and X^{D} is unsubstituted phenylthio. Even more preferably, R₂ is selected from benzyl and 4-methylbenzyl; and R₈^{D} is selected from benzoyl and 4-chlorobenzoyl.

The glycosyl donors of formula **3** can be made in a conventional manner. L-Fucose can be peracetylated, and then the resulting L-fucose tetraacetate can be thiolized with R₁₁SH in the presence of a Lewis acid to give the corresponding thiofucoside. Removal of the acetyl groups can be achieved under Zemplén conditions, and the resulting triol can be treated with dimethoxy-propane/acid to form the 3,4-acetonide. The free OH-group in the 2^{nd} position can then be deprotonated with NaH, and the alcoholate can be reacted with R₂-halogenide to give a compound of formula **3,** wherein X is -SR₁₁, R₂ is as defined above and two R₈ groups together form isopropylidene. The isopropylidene-acetal can be removed by acidic hydrolysis, and the liberated OH-groups can be protected either by means of a mixture of NaH and an (optionally substituted)benzyl-halogenide to yield a compound of formula **3,** wherein X is -SR₁₁, R₂ is as above and R₈ is a group removable by hydrogenolysis, or by acylation with an acyl halogenide or anhydride to form a compound of formula **3,** wherein X is -SR₁₁, R₂ is as above and R₈ is acyl. When the alkylation reaction is carried out with a thiofucoside triol, a compound of formula **3,** wherein R₂ and R₈ are the same, can be obtained. The protected thiofucoside derivative, described above, can be used either as a glycosyl donor in the glycosylation reaction or can be converted to another anomerically activated compound (e.g. wherein X is a halogen or trichloroacetimidate) in a conventional manner.

The acceptors of formula **4** can also be made in a conventional manner. Starting from the known octa-*O*-acetyl lactose or hepta-*O*-acetyl lactosyl bromide, the corresponding R₁-lactoside can be formed with R₁OH under Lewis-acid (e.g. mercury salt or BF₃-etherate) activation. By de-*O*-acetylation (e.g. Zemplén-deprotection, aminolysis or basic hydrolysis) followed by regioselective acetonidation with dimethoxypropane in the presence of an acid catalyst, the 3',4'-protected lactoside can be obtained. This lactoside can then be selectively acylated with an R₄-halogenide or (R₄)₂O anhydride by, e.g., the procedure of Tsukida et al. J. Org. Chem. 62, 6876 (1997), leading to compounds of formula **4** wherein R₁ and R₄ are as defined above, and two R₅ groups together form isopropylidene. The selective acylation according to Tsukida et al. can be performed on R₁-lactoside resulting in the formation of compounds of formula **4** wherein R₄ and R₅ mean identical acyl groups.

The compounds of formulae **2, 2B** and **2C** above represent crucial intermediates in the total synthesis of 3-FL. Some of them are novel. Thus, the fifth aspect of this invention relates to a compound of formula **2A** 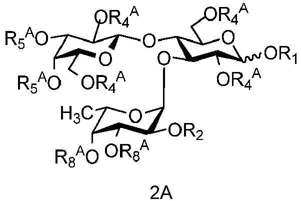 wherein R₁ and R₂ each are as defined above; R₄^{A} is selected from acyl and H; R₅^{A} is selected from acyl and H, or two R₅^{A} groups together form a moiety 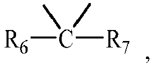 wherein R₆ and R₇ are as defined above; and R₈^{A} is selected from acyl and H, or two R₈^{A} groups together form a moiety 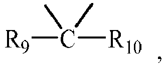 wherein R₉ and R₁₀ are as defined above; provided that R₄^{A}, R₅^{A} and R₈^{A} are not all H;
or a hydrate or solvate thereof.

Each of the novel derivatives of formula **2A** can be considered as a single chemical entity including α and β anomers, as well as an anomeric mixture of α and β isomers. The compounds of formula **2A** can be crystalline solids, oils, syrups, precipitated amorphous material or spray dried products. If crystalline, compounds of formula **2A** could exist either in anhydrous or hydrated crystalline forms, incorporating one or several molecules of water into their crystal structures. Similarly, the compounds of formula **2A** could exist in crystalline forms incorporating ligands such as organic molecules and/or ions into their crystal structures.

Preferably in compounds of formula **2A,** R₁ is selected from benzyl, 4-methylbenzyl, naphthylmethyl, 4-phenylbenzyl, 4-chlorobenzyl, 4-methoxybenzyl, 3,4-dimethoxybenzyl, 2,4,6-trimethylbenzyl and 2,3,4,5,6-pentamethylbenzyl, more preferably from benzyl and 4-methylbenzyl; R₂ is selected from benzyl, 4-methylbenzyl, naphthylmethyl, benzyloxycarbonyl, 4-phenylbenzyl, 4-chlorobenzyl, 4-methoxybenzyl, 3,4-dimethoxybenzyl, 2,4,6-trimethylbenzyl and 2,3,4,5,6-pentamethylbenzyl; R₄^{A} is selected from acyl and H; R₅^{A} is selected from acyl and H, or two R₅^{A} groups together form a moiety 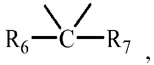 wherein R₆ and R₇ independently are selected from alkyl and phenyl, or R₆ and R₇ together with the carbon atom to which they are attached form a cycloalkylidene; and R₈^{A} is selected from acyl and H. More preferably in compounds of formula **2A,** R₁ and R₂ are independently selected from benzyl and 4-methylbenzyl; R₄^{A} is selected from acetyl, pivaloyl, benzoyl, 4-chlorobenzoyl and H; R₅^{A} is H, or two R₅^{A} groups together form an isopropylidene or a cyclohexylidene; R₈^{A} is selected from acetyl, pivaloyl, benzoyl, 4-chlorobenzoyl and H; and -OR₁ is in β-orientation.

It is believed that this invention provides a process suitable for large scale manufacturing of 3-*O*-fucosyllactose and novel intermediates for its synthesis. The process features the hydrogenolysis of *O*-benzyl/substituted *O*-benzyl moieties of the novel protected 3-*O*-fucosyllactose intermediates. An important advantage of the process is that its novel *O-*benzylated/substituted *O*-benzylated 3-*O*-fucosyllactose intermediates have useful crystalline properties that assist significantly in their purification. In particular, the highly crystalline properties of the 3-*O*-fucosyllactose intermediates of the process allow the chemical process steps to be carried out with only crystallisation procedures for purifying the products and intermediates produced in each step. This allows the chemical process steps to be separated from each other, thereby providing real opportunities for scaling-up and optimizing the individual steps.

Other features of the invention will become apparent from the following Examples which are given for illustration of the invention and not to limit it.

### EXAMPLES

### Example 1. Donors of formula 3

Donors of formula **3** were prepared according to WO 2010/115934 and WO 2010/115935.

### Example 2. Typical procedure for making acceptors of formula 4

Benzyl 3',4'-*O*-isopropylidene-2,6,2',6'-tetra-*O*-benzoyl-β-lactoside Benzyl 3',4'-*O*-isopropylidene-β-lactoside (20 g) was dissolved in pyridine (30 ml). The solution was cooled to 0 °C and a mixture of benzoyl chloride (21 ml) and DCM (40 ml) was added dropwise through a dropping funnel over 6 h. The reaction mixture was stirred for another 2 h at 0 °C and at 5 °C for 24 hours. Methanol (10 ml) was then added and the solvents were removed in vacuo. The remaining residue was redissolved in EtOAc (200 ml) and washed with water (100 ml), sat. NaHCO₃ (100 ml), 2x 1M HCl (100 ml), water (100 ml) and brine (100 ml). After removing the solvent in vacuo, the residue was recrystallized from MeOH (28 g, 72%).

### Example 3. Benzyl 3-O-(2,3,4-tri-O-benzyl-α-L-fucopyranosyl)-3',4'-O-isopropylidene-2,6,2',6'-tetra-O-benzoyl-β-lactoside

To a solution of phenyl 2,3,4-tri-*O*-benzyl-1-thio-β-L-fucopyranoside (133 g) in DCM (439 ml) bromine (16 ml) in DCM (50 ml) was added dropwise at 0 °C over a period of 60 minutes. After addition of the bromine solution the reaction mixture was stirred for additional 15 to 20 minutes. Cyclohexene (35 ml) was then added dropwise, followed by the addition of benzyl 3',4'-*O*-isopropylidene-2,6,2',6'-tetra-*O*-benzoyl-β-lactoside (120 g) and TBAB (8 g) in DCM (330 ml) and DMF (330 ml). The reaction mixture was stirred until TLC (toluene/acetone 12:1) showed completion, then it was diluted with 1.7 1 of EtOAc. The organic layer was washed with sat. Na₂S₂O₃/sat. NaHCO₃ (1:1), sat. NaHCO₃/brine (4:1), water/brine, water/brine/1N HCl (1:1:1), sat. NaHCO₃/brine (2:1), and brine. The organic phase was dried over MgSO₄ and the solvents were removed in vacuo to obtain an orange oil which was recrystallized from EtOAc/hexane (1:3) to obtain 148 g of crystals (84 %).

### Example 4. Benzyl 3-O-(2,3,4-tri-O-benzyl-α-L-fucopyranosyl)-3',4'-O-isopropylidene-β-lactoside

The compound according to example 3 (148 g) was added to a 0.1 M solution of NaOMe in methanol (1.5 1). The suspension was warmed to 40 °C. Complete debenzoylation was confirmed by TLC (toluene/acetone 1:1). H⁺-IR120 Amberlite resin was added to neutralize the solution and the methanol was removed in vacuo. The residue was redissolved in EtOAc (1350 ml) and extracted with water (900 ml) 0.5 N HCl (900 ml), sat. NaHCO₃ (900 ml) and brine (450 ml). The solvent was removed in vacuo and the product was crystallised from EtOAc/hexane (1:2) to yield 79 g of product (79 %).
M.p.: 101-103 °C.

### Example 5. Benzyl 3-O-(2,3,4-tri-O-benzyl-α-L-fucopyranosyl)-β-lactoside

The compound according to example 4 (79 g) was dissolved in DCM (400 ml), MeOH (280 ml) and water (40 ml). TFA (80 ml) was then added slowly at room temperature. After the addition is completed, the temperature was raised to 40 °C. The progress of the reaction was followed by TLC (toluene/acetone 1:2). When no starting material could be detected, the reaction was cooled down in an ice-bath to 0 °C. Slowly and portionwise 500 ml of sat. NaHCO₃ solution was added followed by EtOAc (1.2 1) together with additional 250 ml of sat. NaHCO₃ solution and 250 ml of brine. The organic layer was extracted two more times with 500 ml of sat. NaHCO₃ solution and 500 ml of brine. The solvent was removed in vacuo and the residue was crystallised from EtOAc/Et₂O (2:3) to yield 60 g of product (80 %). ¹H-NMR (CD₃OD) δ (ppm): 1.18 (d, J=6.1Hz, 3H); 3.34-3.56 (m, 4H); 3.57-3.69 (m, 2H); 3.78 (m, 2H); 3.95 (m, 7H); 4.1 (dd, J=2.9Hz, J=10.1Hz, 1H); 4.4 (d, J=7.6Hz, 1H); 4.44 (d, J=7Hz, 1H); 4.57 (d, J=11.0Hz, 1H); 4.65 (d, H=11.7Hz, 1H); 4.69 (d, 11.7Hz, 1H); 4.81 (m, 1H); 4.93 (m, 3H); 5.7 (d, J=3.96Hz, 1H); 7.15-7.57 (m, 20H). ¹³C-NMR (CD₃OD) δ (ppm): 15.75, 60.17, 60.18, 62.34, 66.43, 69.00, 70.77, 71.78, 71.93, 72.82, 73.65, 75.25, 75.55, 75.93, 76.14, 76.28, 77.57, 78.90, 78.94, 78.97, 97.09, 102.38, 102.58, 127.22, 127.28, 127.32, 127.37, 127.51, 127.62, 127.98, 128.07, 128.11, 128.12, 128.16, 128.45, 137.78, 138.50, 139.11, 139.42. M.p.: 123-125 °C.

### Example 6. Benzyl 3-O-(3,4-di-O-benzoyl-2-O-benzyl-α-L-fucopyranosyl)-3',4'-O-isopropylidene-2,6,2',6'-tetra-O-benzoyl-β-lactoside

Benzyl 3',4'-*O*-isopropylidene-2,6,2',6'-tetra-*O*-benzoyl-β-lactoside (14.5 g), phenyl 3,4-di-*O*-benzoyl-2-*O*-benzyl-1-thio-β-L-fucopyranoside (12.4 g) and NBS (4.38 g) were dissolved in toluene (300 ml). The mixture was cooled in an ice-bath to 0-5 °C. TMSOTf (310 µl) was added and the mixture was stirred for 10 minutes. The reaction was stopped with adding an aqueous Na₂S₂O₃/NaHCO₃-solution. After separation of the phases the organic phase was washed with water, dried (Na₂SO₄) and concentrated to dryness. The residue was crystallized from ethanol. As soon as the solution became lukewarm the solid was filtered off, washed 2 times with ethanol and dried to yield 20 g of product (92 %). ¹H-NMR (CDCl₃) δ (ppm): 1.37 (m, 6H); 1.66 (s, 3H); 3.56 (m, 1H); 4.00 (m, 2H); 4.1-4.3 (m, 5H); 4.57-4.79 (m, 3H); 5.0 (m, 1H); 5.3 (m, 2H); 5.55 (m, 2H); 5.74 (d, J= 3.1Hz, 1H); 5.87 (dd, J=10.6 Hz, J=3.7 Hz, 1H); 6.79 (m, 2H); 6.92 (m, 2H); 6.99-7.23 (m, 8H); 7.32-7.6 (m, 16H); 7.66 (m, 2H); 7.85 (m, 2H); 7.96 (m, 2H); 8.11 (m, 4H); 8.26 (m, 2H). ¹³C-NMR (CDCl₃) δ (ppm): 54.66, 62.55, 63.129, 65.165, 70.28, 70.87, 72.14, 72.39, 72.43, 73.1, 73.59, 73.78, 74.86, 75.00, 75.10, 97.43, 99.26, 100.69, 111.30, 128.00, 128.15, 128.20, 128.47, 128.54, 128.84, 128.87, 129.00, 129.661, 129.84, 129.98, 130.10, 130.20, 130.29, 132.74, 133.18, 133.41, 133.58, 133.70, 133.84, 136.74, 137.65, 164.81, 165.06, 165.09, 166.20, 166.27, 166.83. M.p.: 189 °C.

### Example 7. According to example 6 the following protected trisaccharides were synthesized from the appropriate donors and acceptors:

### a) benzyl 3-O-(3,4-di-O-acetyl-2-O-benzyl-α-L-fucopyranosyl)-3',4'-O-isopropylidene-2,6,2',6'-tetra-O-benzoyl-β-lactoside

¹H-NMR (CDCl₃) δ (ppm): 1.28 (m, 6H); 1.58 (s, 3H); 1.78(s, 3H); 2.03(s, 3H); 3.48 (m, 1H); 3.67 (dd, J=3.8Hz, J=10.7Hz, 1H); 3.87 (m, 1H); 4.02-4.27 (m, 6H);4.32-4.57 (m, 6H); 4.63 (m, 1H); 4.71 (d, J=12.2Hz, 1H); 4.81 (dd, J=8.3Hz, J=12.3, 1H); 4.96 (dd, J=4.2Hz, J=11.9Hz); 5.13 (dd, J=6.0Hz, J=12.9Hz, 1H); 5.24(dd, J=7.3Hz, J=8.6Hz, 1H); 5.34(m, 1H); 5.38-5.5 (m, 2H): 6.84-7.70 (m, 24H); 7.86-8.24 (m, 8H). ¹³C-NMR (CDCl₃) δ (ppm): 16.36, 20.90, 20.94, 26.35, 28.01, 54.66, 62.47, 62.89, 64.62, 70.28, 70.53, 72.25, 72.28, 72.66, 72.81, 73.51, 73.67, 74.78, 74.86, 75.12, 97.61, 99.24, 100.57, 111.21, 127.84, 128.14, 128.44, 128.82, 128.91, 128.98, 129.60, 130.07, 130.13, 130.17, 133.47, 133.58, 133.64, 133.80, 136.72, 138.06, 164.65, 165.04, 166.21, 166.76, 169.76, 170.72. M.p.: 189 °C.

### b) benzyl 3-O-(3,4-di-O-(4-chlorobenzoyl)-2-O-benzyl-α-L-fucopyranosyl)-3',4'-O-isopropylidene-2,6,2',6'-tetra-O-benzoyl-β-lactoside

¹H-NMR (CDCl₃) δ (ppm): 1.26 (m, 6H); 1.54 (s, 3H); 3.48 (m, 1H); 3.83 (m, 2H); 3.98 (d, J=11.9Hz, 1H); 4.08-4.24 (m, 4H); 4.30-4.45 (m, 4H); 4.47-4.70 (m, 3H); 4.80-5.0 (m, 2H); 5.21 (m, 2H); 5.45(m, 2H); 5.58(m, 1H); 5.72 (dd, J=10.6Hz, J=3.4Hz, 1H): 6.64 (m, 2H); 6.79-7.12 (m, 10H); 7.22-7.62 (m, 16H); 7.66 (m, 2H); 7.86 (m, 2H); 8.02 (m, 4H); 8.15 (m, 2H). ¹³C-NMR (CDCl₃) δ (ppm): 16.48, 26.42, 28.11, 54.67, 62.51, 63.06, 64.98, 70.28, 70.93, 72.03, 72.26, 72.38, 73.29, 73.54, 73.69, 73.75, 74.84, 74.97, 75.11, 97.21, 99.19, 100.67, 111.27, 128.00, 128.12, 128.17, 128.47, 128.52, 128.55, 128.83, 128.87, 128.96, 128.99, 129.65, 130.09, 130.16, 130.27, 131.15, 131.30, 133.45, 133.60, 133.75, 133.84, 136.69, 137.55, 139.23, 139.77, 164.17, 164.79, 165.06, 165.31, 166.23, 169.75. M.p.: 186-187 °C.

### c) benzyl 3-O-(3,4-di-O-acetyl-2-O-benzyl-α-L-fucopyranosyl)-3',4'-O-isopropylidene-2,6,2',6'-tetra-O-acetyl-β-lactoside

¹H-NMR (CDCl₃) δ (ppm): 1.13 (d, J=6.3Hz, 3H); 1.30 (s, 3H); 1.50 (s, 3H), 1.88-2.14 (6s, 18H); 3.47 (m, 1H); 3.81-4.00(m, 4H); 4.05-4.20 (m, 3H); 4.27 (d, J=11.8Hz, 1H); 4.37-4.66 (m, 7H); 4.78-4.94 (m, 3H); 5.15 (t, 7.8Hz, 1H); 5.24-5.34 (m, 3H); 7.2-7.35 (m, 10H). M.p.: 185 °C.

### d) benzyl 3-O-(3,4-di-O-(4-chlorobenzoyl)-2-O-benzyl-α-L-fucopyranosyl)-3',4'-O-isopropyfidene-2,6,2',6'-tetra-O-(4-chlorobenzoyl)-β-lactoside

¹H-NMR (CDCl₃) δ (ppm): 1.26 (m, 6H); 1.52 (s, 3H); 3.44 (m, 1H); 3.76 (m, 2H); 3.92 (m, 1H); 4.09-4.22 (m, 4H); 4.29-4.42 (m, 4H); 4.45-4.69 (m, 3H); 4.78-5.0 (m, 2H); 5.13 (m, 2H); 5.38(m, 2H); 5.60(m, 1H); 5.72 (dd, J=10.2Hz, J=3.6Hz, 1H): 6.84-8.12 (m, 34H).

### e) benzyl 3-O-(3,4-di-O-pivaloyl-2-O-benzyl-α-L-fucopyranosyl)-3',4'-O-isopropylidene-2,6,2',6'-tetra-O-pivaloyl-β-lactoside

¹H-NMR (CDCl₃) δ (ppm): 0.8-1.57 (m, 63H); 3.48 (m, 1H); 3.78-3.98 (m, 3H); 4.06 (m, 3H); 4.15-4.33 (m, 2H); 4.37-4.62 (m, 7H); 4.80 (d, J=11.7Hz, 1H); 5.1 (dd, J=7.7Hz, J=8.0Hz, 1H); 5.29 (dd, J=3.2Hz, J=10.2Hz, 1H); 5.37 (m, 2H); 7.26 (m, 10H). ¹³C-NMR (CDCl₃) δ (ppm): 14.36, 22.92, 27.19, 27.21, 27.24, 27.39, 27.43, 27.45, 27.51, 27.54, 27.57, 32.11, 38.85, 38.95, 38.96, 39.29, 54.66, 61.99, 62.84, 64.95, 70.59, 70.99, 71.67, 71.79, 72.67, 72.84, 73.64, 73.72, 74.20, 74.52, 74.66, 74.85, 97.12, 100.03, 100.22, 110.04, 127.50, 127.90, 128.00, 128.06, 128.22, 128.34, 128.34, 128.52136.83, 138.08, 176.46, 176.65, 176.75, 177.59, 178.15, 178.65.

### Example 8. Benzyl 3-O-(3,4-di-O-benzoyl-2-O-benzyl-α-L-fucopyranosyl)-2,6,2',6'-tetra-O-benzoyl-β-lactoside

To a solution of benzyl 3-*O*-(3,4-di-*O*-benzoyl-2-*O*-benzyl-α-L-fucopyranosyl)-3',4'-*O-*isopropylidene-2,6,2',6'-tetra-*O*-benzoyl-β-lactoside (7.30 g) in 70 ml of DCM, 6 ml of aqueous HClO₄-solution were added. After stirring for 2 h at room temperature the reaction was stopped by adding 40 ml of 10 % NaHCO₃-solution. The organic layer was washed with water, dried (Na₂SO₄), concentrated. Crystallization from ethanol furnished 6.33 g (93 %) of colourless solid. ¹H-NMR (CDCl₃) δ (ppm): 1.24 (d, J=6.3Hz, 3H); 3.56 (m, 3H); 3.90 (m, 2H); 3.99-4.3 (m, 3H); 4.4 (m, 4H); 4.57-4.71 (m, 3H); 4.75 (dd, J=6.6Hz, J=11.9Hz, 1H); 4.95 (dd, J=6.7Hz, J=11.4Hz, 1H); 5.1-5.3 (m, 2H); 5.4 (d, J=6.7Hz, 1H); 5.47 (dd, J=8.4Hz, J=9.1Hz, 1H); 5.67 (m, 2H); 6.7-8.1 (m, 40H). ¹³C-NMR (CDCl₃) δ (ppm): 16.25, 54.66 62.38, 62.77, 65.23, 68.36, 70.26, 71.36, 71.98, 72.13, 72.75, 72.91, 73.67, 73.78, 74.90, 97.30, 99.37, 100.44, 128.02, 128.19, 128.47, 128.80, 128.97, 129.94, 130.00, 130.16, 133.06, 133.22, 133.53, 133.67, 133.76, 136.79, 137.58, 164.81, 165.94, 166.00, 166.30, 166.38, 166.87. M.p.: 123-125 °C.

### Example 9. Benzyl 3-O-(2-O-benzyl-α-L-fucopyranosyl)-3',4'-O-isopropylidene-β-lactoside

Benzyl 3-*O*-(3,4-di-*O*-benzoyl-2-*O*-benzyl-α-L-fucopyranosyl)-2,6,2',6'-tetra-*O*-benzoyl-β-lactoside (10.0 g) was dissolved in 100 ml of a 0.1 M NaOMe/MeOH solution and stirred at room temperature for 48 h. Methanol was then removed and the residue was taken up in a EtOAc/H₂O 1:1 mixture. The aqueous phase was extracted twice with EtOAc. The combined organic phases were dried (Na₂SO₄) and concentrated to yield 3.41 g (64 %) of colourless solid. ¹H-NMR (CD₃OD) δ (ppm): 1.15 (d, J=6.3Hz, 3H); 1.29 (s, 3H); 1.43 (s, 3H), 3.35 (m, 2H); 3.51-3.72 (m, 5H); 3.79 (m, 3H); 3.88-4.16 (m, 5H); 4.38 (m, 2H); 4.65 (m, 2H); 4.76 (m, 1H); 4.91 (m, 1H); 5.72 (d, J=3.8Hz, 1H); 7.18-7.50 (m, 10H). ¹³C-NMR (D₃COD) δ (ppm): 16.65, 25.35, 27.67, 51.51, 59.99, 61.68, 65.68, 68.90, 70.76, 71.51, 72.93, 73.66, 73.88, 74.00, 74.30, 75.70, 75.79, 76.08, 76.67, 79.67, 96.47, 102.09, 102.27, 109.70, 127.54, 127.63, 128.10, 128.14, 128.18, 128.38, 128.44, 129.32, 133.8, 137.77, 138.46. M.p.: 98-100 °C.

### Example 10. Benzyl 3-O-(2-O-benzyl-α-L-fucopyranosyl)-β-lactoside

Benzyl 3-*O*-(3,4-di-*O*-benzoyl-2-*O*-benzyl-α-L-fucopyranosyl)-2,6,2',6'-tetra-*O*-benzoyl-β-lactoside (5.73 g) was suspended in 30 ml of a 1 M NaOMe/MeOH-solution. After stirring for 2.5 h at room temperature the clear solution was neutralized with Amberlite IR-120 H⁺ resin. The resin was filtered off and washed with methanol. The solvent was removed and the residue was taken up in H₂O/DCM 1:1 mixture. The aqueous phase was concentrated, and coevaporated with toluene. Crystallization in diethyl ether/methanol afforded the product as a colourless solid (2.40 g, 81 %). ¹H-NMR (D₃COD) δ (ppm): 1.1 (d, J=6.9Hz, 3H); 3.27-3.44 (m, 4H); 3.48-3.63 (m, 3H); 3.65-3.79 (m, 5H); 3.79-3.94 (m, 3H); 4.00 (dd, J=10.0Hz, J=3.4Hz, 1H); 4.33 (m, 2H); 4.60 (m, 2H); 5.63 (d, J=3.8Hz, 1H); 7.1-7.3 (m, 6H); 7.33-7.45 (m, 4H). ¹³C-NMR (D₃COD) δ (ppm): 16.55, 63.141, 67.104, 70.06, 71.94, 72.58, 72.95, 73.80, 74.44, 74.84, 76.84, 76.97, 77.43, 78.16, 97.76, 103.50, 103.89, 129.30, 129.35, 129.58, 138.97, 139.69. M.p.: 150 °C.

### Example 11. 3-O-Fucosyl lactose

A) Benzyl 3-*O*-(2,3,4-tri-*O*-benzyl-α-L-fucopyranosyl)-β-lactoside (3.8 g) was dissolved in a mixture of isopropanol-methanol (1:2, 60 ml). 10 % Pd on charcoal (0.23 g) was added and the mixture was stirred under H₂-atmosphere (20 bar in an autoclave) at 40 °C for 24 hours. The precipitated product was dissolved by adding small amount of water and few drops of acetic acid, and the hydrogenolysis was continued for 8 hours. The catalyst was filtered off and the solvents removed. The product was dried in vacuo and precipitated by adding propanol (practically quantitative yield).
B) Benzyl 3-*O*-(2-*O*-benzyl-α-L-fucopyranosyl)-β-lactoside (1.0 g) was dissolved in a mixture of MeOH, water and AcOH (3+3+0.4 ml). 10 % Pd on charcoal (100 mg) was added and the mixture was stirred under H₂-atmosphere (10 bar in an autoclave) at 35 °C for 10 hours. The catalyst was filtered off and the solvents removed. The product was dried in vacuo and precipitated to a fine white powder as follows: a solution of the crude material in water/MeOH (2/1) was dropped into a 50 °C mixture of acetone/MeOH 2/1. The mixture was allowed to cool to room temperature and was stirred at 5 °C for 24 hours to yield 3-FL (690 mg, 95 %). ¹H-NMR (D₂O) δ (ppm): 1.0 (d, J=7Hz, 3H); 3.23-3.34 (m, 2H); 3.35-3.51 (m, 3H); 3.52-3.74 (m, 9H); 3.74-3.83 (m, 2H); 4.24 (d, J=7.9Hz, 1H); 4.46 (d, J=7.9Hz, 0.52H); 5.00 (d, J=3.7Hz, 0.43H); 5.19 (d, J=4Hz, 0.43H); 5.25 (d, J=4.1Hz, 0.57H). ¹³C-NMR (D₂O) δ (ppm): 15.34, 59.78, 59.87, 61.62, 61.66, 66.57, 66.61, 68.13, 68.16, 68.43, 69.32, 69.38, 71.01, 71.24, 72.06, 72.50, 72.69, 72.75, 72.78, 74.80, 75.06, 75.47, 75.63, 77.09, 92.19, 95.92, 98.48, 98.61, 101.90. HPLC purity: 95-98 %.

## Claims

1. A compound of formula **1** 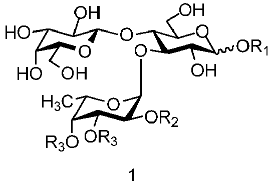 wherein R₁ and R₂ are independently a group removable by hydrogenolysis; and R₃ is selected from a group removable by hydrogenolysis and H;
or a hydrate or solvate thereof.

2. The compound of claim 1, wherein R₁ is selected from benzyl, 4-methylbenzyl, naphthylmethyl, 4-phenylbenzyl, 4-chlorobenzyl, 4-methoxybenzyl, 3,4-dimethoxybenzyl, 2,4,6-trimethylbenzyl and 2,3,4,5,6-pentamethylbenzyl; R₂ is selected from benzyl, 4-methylbenzyl, naphthylmethyl, benzyloxycarbonyl, 4-phenylbenzyl, 4-chlorobenzyl, 4-methoxybenzyl, 3,4-dimethoxybenzyl, 2,4,6-trimethylbenzyl and 2,3,4,5,6-pentamethylbenzyl; and R₃ is selected from benzyl, 4-methylbenzyl, naphthylmethyl, benzyloxycarbonyl, 4-phenylbenzyl, 4-chlorobenzyl, 4-methoxybenzyl, 3,4-dimethoxybenzyl, 2,4,6-trimethylbenzyl, 2,3,4,5,6-pentamethylbenzyl and H.

3. The compound of claim 2, wherein R₁ and R₂ are independently selected from benzyl and 4-methylbenzyl; R₃ is selected from benzyl, 4-methylbenzyl and H; and -OR₁ is in β-orientation.

4. A method for making 3-*O*-fucosyllactose comprising the step of subjecting to hydrogenolysis a compound of formula **1** 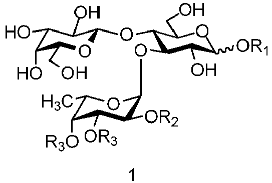 wherein R₁ and R₂ are independently a group removable by hydrogenolysis; and R₃ is selected from a group removable by hydrogenolysis and H;
or a hydrate or solvate thereof.

5. The method of claim 4, wherein the hydrogenolysis is carried out in water, in one or more C₁-C₆ alcohols or in a mixture of water and one or more C₁-C₆ alcohols, in the presence of a palladium or a Raney nickel catalyst.

6. The method of claim 5, wherein said palladium is palladium on charcoal or palladium black.

7. The method of any of the claims 4-6, wherein in the compound of formula **1,** R₁ is selected from benzyl, 4-methylbenzyl, naphthylmethyl, 4-phenylbenzyl, 4-chlorobenzyl, 4-methoxybenzyl, 3,4-dimethoxybenzyl, 2,4,6-trimethylbenzyl and 2,3,4,5,6-pentamethylbenzyl; R₂ is selected from benzyl, 4-methylbenzyl, naphthylmethyl, benzyloxycarbonyl, 4-phenylbenzyl, 4-chlorobenzyl, 4-methoxybenzyl, 3,4-dimethoxybenzyl, 2,4,6-trimethylbenzyl and 2,3,4,5,6-pentamethylbenzyl; and R₃ is selected from benzyl, 4-methylbenzyl, naphthylmethyl, benzyloxycarbonyl, 4-phenylbenzyl, 4-chlorobenzyl, 4-methoxybenzyl, 3,4-dimethoxybenzyl, 2,4,6-trimethylbenzyl, 2,3,4,5,6-pentamethylbenzyl and H.

8. The method of claim 7, wherein R₁ and R₂ are independently selected from benzyl and 4-methylbenzyl; R₃ is selected from benzyl, 4-methylbenzyl and H; and -OR₁ is in β-orientation.

9. The method of any of claims 4 to 8 further comprising the prior step of:
- converting a compound of formula **2** 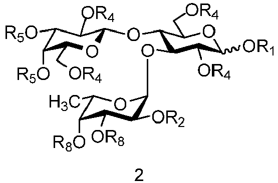 wherein R₁ and R₂ each are as defined above; R₄ is acyl; R₅ is acyl, or two R₅ groups together form a moiety 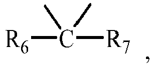 wherein R₆ and R₇ are independently selected from alkyl and phenyl, or wherein R₆ and R₇ together with the carbon atom to which they are attached form a cycloalkylidene; and R₈ is selected from a group removable by hydrogenolysis and acyl, or two R₈ groups together form a moiety 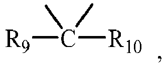 wherein R₉ and R₁₀ independently are selected from alkyl and phenyl, or wherein R₉ and R₁₀ together with the carbon atom to which they are attached form cycloalkylidene,
into a compound of formula **1** or a hydrate or solvate thereof by:
a) deacylating the R₄ acyl groups and any R₅ and R₈ acyl groups of the compound of formula **2;** and optionally
b) removing any moiety 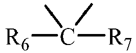 and any moiety 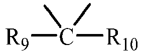 from the compound of formula **2** by treatment with an acid.

10. The method according to claim 9, wherein R₄ is selected from acetyl, benzoyl and 4-chlorobenzoyl, two R₅ groups together form isopropylidene or cyclohexylidene and R₈ is selected from benzyl, 4-methylbenzyl, naphthylmethyl, benzyloxycarbonyl, 4-phenylbenzyl, 4-chlorobenzyl, 4-methoxybenzyl, 3,4-dimethoxybenzyl, 2,4,6-trimethylbenzyl, 2,3,4,5,6-pentamethylbenzyl, acetyl, pivaloyl, benzoyl and 4-chlorobenzoyl, preferably benzyl, 4-methylbenzyl, acetyl, pivaloyl, benzoyl and 4-chlorobenzoyl.

11. A method for making a compound of formula **1** 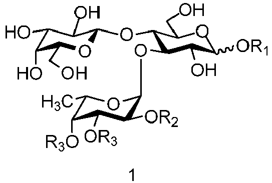 wherein R₁ and R₂ are independently a group removable by hydrogenolysis, and R₃ is selected from a group removable by hydrogenolysis and H; or a hydrate or solvate thereof, from a compound of formula **2** 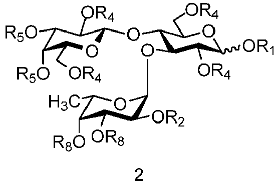 wherein R₁ and R₂ each are as defined above; R₄ is acyl; R₅ is acyl, or two R₅ groups together form a moiety 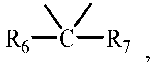 wherein R₆ and R₇ are independently selected from alkyl and phenyl, or wherein R₆ and R₇ together with the carbon atom to which they are attached form a cycloalkylidene; and R₈ is selected from a group removable by hydrogenolysis and acyl, or two R₈ groups together form a moiety 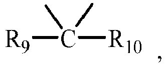 wherein R₉ and R₁₀ independently are selected from alkyl or phenyl, or wherein R₉ and R₁₀ together with the carbon atom to which they are attached form a cycloalkylidene,
comprising the steps of:
a) removing, by deacylation, the R₄ acyl groups and any R₅ and R₈ acyl groups; and optionally
b) removing any moiety 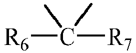 and any moiety 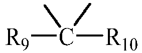 by treatment with an acid.

12. The method according to claim 11, wherein R₁ is selected from benzyl, 4-methylbenzyl, naphthylmethyl, 4-phenylbenzyl, 4-chlorobenzyl, 4-methoxybenzyl, 3,4-dimethoxybenzyl, 2,4,6-trimethylbenzyl and 2,3,4,5,6-pentamethylbenzyl; R₂ is selected from benzyl, 4-methylbenzyl, naphthylmethyl, benzyloxycarbonyl, 4-phenylbenzyl, 4-chlorobenzyl, 4-methoxybenzyl, 3,4-dimethoxybenzyl, 2,4,6-trimethylbenzyl and 2,3,4,5,6-pentamethylbenzyl; R₃ is selected from benzyl, 4-methylbenzyl, naphthylmethyl, benzyloxycarbonyl, 4-phenylbenzyl, 4-chlorobenzyl, 4-methoxybenzyl, 3,4-dimethoxybenzyl, 2,4,6-trimethylbenzyl, 2,3,4,5,6-pentamethylbenzyl and H; R₄ is selected from acetyl, benzoyl and 4-chlorobenzoyl; two R₅ groups together form isopropylidene or cyclohexylidene and R₈ is selected from benzyl, 4-methylbenzyl, naphthylmethyl, benzyloxycarbonyl, 4-phenylbenzyl, 4-chlorobenzyl, 4-methoxybenzyl, 3,4-dimethoxybenzyl, 2,4,6-trimethylbenzyl, 2,3,4,5,6-pentamethylbenzyl, acetyl, pivaloyl, benzoyl and 4-chlorobenzoyl.

13. The method according to claim 12, wherein R₁ and R₂ are independently selected from benzyl and 4-methylbenzyl; R₃ is selected from benzyl, 4-methylbenzyl and H and R₈ is selected from benzyl, 4-methylbenzyl, acetyl, pivaloyl, benzoyl and 4-chlorobenzoyl.

14. A compound of formula **2A** 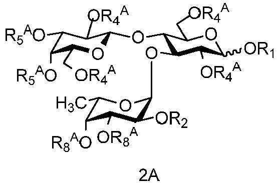 wherein R₁ and R₂ are independently a group removable by hydrogenolysis; R₄^{A} is selected from acyl and H; R₅^{A} is selected from acyl and H, or two R₅^{A} groups together form a moiety 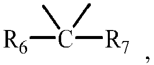 wherein R₆ and R₇ are independently selected from alkyl and phenyl, or wherein R₆ and R₇ together with the carbon atom to which they are attached form a cycloalkylidene; and R₈^{A} is selected from acyl and H, or two R₈^{A} groups together form a moiety 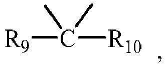 wherein R₉ and R₁₀ independently are selected from alkyl or phenyl, or wherein R₉ and R₁₀ together with the carbon atom to which they are attached form a cycloalkylidene, provided that R₄^{A}, R₅^{A} and R₈^{A} are not all H;
or a hydrate or solvate thereof.

15. The compound according to claim 14, wherein R₁ is selected from benzyl, 4-methylbenzyl, naphthylmethyl, 4-phenylbenzyl, 4-chlorobenzyl, 4-methoxybenzyl, 3,4-dimethoxybenzyl, 2,4,6-trimethylbenzyl and 2,3,4,5,6-pentamethylbenzyl, preferably benzyl and 4-methylbenzyl; and R₂ is selected from benzyl, 4-methylbenzyl, naphthylmethyl, benzyloxycarbonyl, 4-phenylbenzyl, 4-chlorobenzyl, 4-methoxybenzyl, 3,4-dimethoxybenzyl, 2,4,6-trimethylbenzyl and 2,3,4,5,6-pentamethylbenzyl, preferably benzyl and 4-methylbenzyl; R₄^{A} is selected from acetyl, pivaloyl, benzoyl, 4-chlorobenzoyl and H; R₅^{A} is H, or two R₅^{A} groups together form an isopropylidene or a cyclohexylidene; R₈^{A} is selected from acetyl, pivaloyl, benzoyl, 4-chlorobenzoyl and H; and -OR₁ is in β-orientation.

## Patentansprüche

1. Verbindung mit der Formel 1 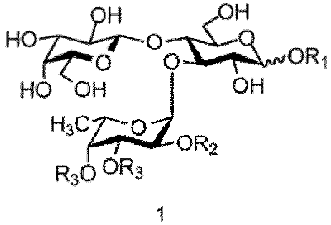 wobei R₁ und R₂ unabhängig für eine Gruppe stehen, die durch Hydrogenolyse entfernbar ist, und R₃ aus einer Gruppe, die durch Hydrogenolyse entfernbar ist, und aus H ausgewählt ist ,
oder ein Hydrat oder Solvat davon.

2. Verbindung nach Anspruch 1, wobei R₁ aus Benzyl, 4-Methylbenzyl, Naphthylmethyl, 4-Phenylbenzyl, 4-Chlorbenzyl, 4-Methoxybenzyl, 3,4-Dimethoxybenzyl, 2,4,6-Trimethylbenzyl und 2,3,4,5,6-Pentamethylbenzyl ausgewählt ist, R₂ aus Benzyl, 4-Methylbenzyl, Naphthylmethyl, Benzyloxycarbonyl, 4-Phenylbenzyl, 4-Chlorbenzyl, 4-Methoxybenzyl, 3,4-Dimethoxybenzyl, 2,4,6-Trimethylbenzyl und 2,3,4,5,6-Pentamethylbenzyl ausgewählt ist und R₃ aus Benzyl, 4-Methylbenzyl, Naphthylmethyl, Benzyloxycarbonyl, 4-Phenylbenzyl, 4-Chlorbenzyl, 4-Methoxybenzyl, 3,4-Dimethoxybenzyl, 2,4,6-Trimethylbenzyl, 2,3,4,5,6-Pentamethylbenzyl und H ausgewählt ist.

3. Verbindung nach Anspruch 2, wobei R₁ und R₂ unabhängig aus Benzyl und 4-Methylbenzyl ausgewählt sind, R₃ aus Benzyl, 4-Methylbenzyl und H ausgewählt ist und -OR₁ sich in β-Stellung befindet.

4. Verfahren zum Herstellen von 3-O-Fucosyllactose, welches eine Hydrogenolyse einer Verbindung mit der Formel 1 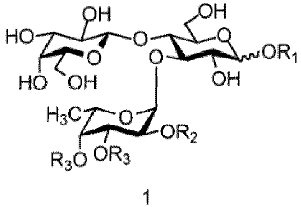 umfasst,
wobei R₁ und R₂ unabhängig für eine Gruppe stehen, die durch Hydrogenolyse entfernbar ist, und R₃ aus einer Gruppe, die durch Hydrogenolyse entfernbar ist, und aus H ausgewählt ist,
oder ein Hydrat oder Solvat davon.

5. Verfahren nach Anspruch 4, wobei die Hydrogenolyse in Wasser, in einem oder mehreren C₁-C₆-Alkoholen oder in einem Gemisch aus Wasser und einem oder mehreren C₁-C₆-Alkoholen im Beisein eines Palladium- oder eines Raney-Nickel-Katalysators ausgeführt wird.

6. Verfahren nach Anspruch 5, wobei es sich bei dem Palladium um Palladium auf Kohle oder Palladiumschwarz handelt.

7. Verfahren nach einem der Ansprüche 4 bis 6, wobei in der Verbindung mit der Formel 1 R₁ aus Benzyl, 4-Methylbenzyl, Naphthylmethyl, 4-Phenylbenzyl, 4-Chlorbenzyl, 4-Methoxybenzyl, 3,4-Dimethoxybenzyl, 2,4,6-Trimethylbenzyl und 2,3,4,5,6-Pentamethylbenzyl ausgewählt ist, R₂ aus Benzyl, 4-Methylbenzyl, Naphthylmethyl, Benzyloxycarbonyl, 4-Phenylbenzyl, 4-Chlorbenzyl, 4-Methoxybenzyl, 3,4-Dimethoxybenzyl, 2,4,6-Trimethylbenzyl und 2,3,4,5,6-Pentamethylbenzyl ausgewählt ist und R₃ aus Benzyl, 4-Methylbenzyl, Naphthylmethyl, Benzyloxycarbonyl, 4-Phenylbenzyl, 4-Chlorbenzyl, 4-Methoxybenzyl, 3,4-Dimethoxybenzyl, 2,4,6-Trimethylbenzyl, 2,3,4,5,6-Pentamethylbenzyl und H ausgewählt ist.

8. Verfahren nach Anspruch 7, wobei R₁ und R₂ unabhängig aus Benzyl und 4-Methylbenzyl ausgewählt sind, R₃ aus Benzyl, 4-Methylbenzyl und H ausgewählt ist und -OR₁ sich in β-Stellung befindet.

9. Verfahren nach einem der Ansprüche 4 bis 8, ferner den folgenden vorhergehenden Schritt umfassend:
- Überführen einer Verbindung mit der Formel 2 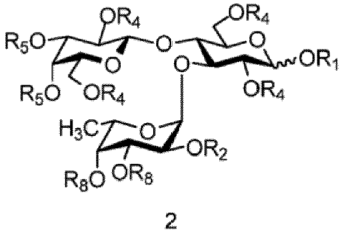 wobei R₁ und R₂ jeweils der vorstehenden Definition entsprechen, es sich bei R₄ um Acyl handelt, es sich bei R₅ um Acyl handelt oder zwei R₅-Gruppen zusammen eine Einheit 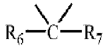 bilden, wobei R₆ und R₇ unabhängig aus Alkyl und Phenyl ausgewählt sind oder wobei R₆ und R₇ zusammen mit dem Kohlenstoffatom, an dem sie gebunden sind, ein Cycloalkyliden bilden und R₈ aus einer Gruppe, die durch Hydrogenolyse entfernbar ist, und Acyl ausgewählt ist oder zwei R₈-Gruppen zusammen eine Einheit 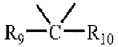 bilden, wobei R₉ und R₁₀ unabhängig aus Alkyl und Phenyl ausgewählt sind oder wobei R₉ und R₁₀ zusammen mit dem Kohlenstoffatom, an dem sie gebunden sind, ein Cycloalkyliden bilden,
zu einer Verbindung mit der Formel 1 oder einem Hydrat oder Solvat davon durch:
a) Deacylieren der R₄-Acylgruppen und jeglicher R₅- und R₈-Acylgruppen der Verbindung mit der Formel 2 und wahlweise
b) Entfernen von jeglicher Einheit 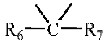 und von jeglicher Einheit 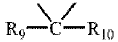 aus der Verbindung mit der Formel 1 durch Behandlung mit einer Säure.

10. Verfahren nach Anspruch 9, wobei R₄ aus Acetyl, Benzoyl und 4-Chlorbenzoyl ausgewählt ist, zwei R₅-Gruppen zusammen Isopropyliden oder Cyclohexyliden bilden und R₈ aus Benzyl, 4-Methylbenzyl, Naphthylmethyl, Benzyloxycarbonyl, 4-Phenylbenzyl, 4-Chlorbenzyl, 4-Methoxybenzyl, 3,4-Dimethoxybenzyl, 2,4,6-Trimethylbenzyl, 2,3,4,5,6-Pentamethylbenzyl, Acetyl, Pivaloyl, Benzoyl und 4-Chlorbenzoyl, vorzugsweise aus Benzyl, 4-Methylbenzyl, Acetyl, Pivaloyl, Benzoyl und 4-Chlorbenzoyl, ausgewählt ist.

11. Verfahren zum Herstellen einer Verbindung mit der Formel 1 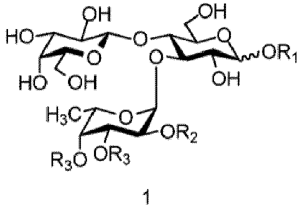 wobei R₁ und R₂ unabhängig für eine Gruppe stehen, die durch Hydrogenolyse entfernbar ist, und R₃ aus einer Gruppe, die durch Hydrogenolyse entfernbar ist, und aus H ausgewählt ist, oder ein Hydrat oder Solvat davon, aus einer Verbindung mit der Formel 2 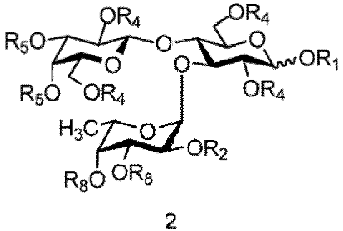 wobei R₁ und R₂ jeweils der vorstehenden Definition entsprechen, es sich bei R₄ um Acyl handelt, es sich bei R₅ um Acyl handelt oder zwei R₅-Gruppen zusammen eine Einheit 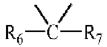 bilden, wobei R₆ und R₇ unabhängig aus Alkyl und Phenyl ausgewählt sind oder wobei R₆ und R₇ zusammen mit dem Kohlenstoffatom, an dem sie gebunden sind, ein Cycloalkyliden bilden und R₈ aus einer Gruppe, die durch Hydrogenolyse entfernbar ist, und Acyl ausgewählt ist oder zwei R₈-Gruppen zusammen eine Einheit 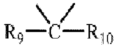 bilden, wobei R₉ und R₁₀ unabhängig aus Alkyl und Phenyl ausgewählt sind oder wobei R₉ und R₁₀ zusammen mit dem Kohlenstoffatom, an dem sie gebunden sind, ein Cycloalkyliden bilden,
umfassend die Schritte:
a) Entfernen der R₄-Acylgruppen und jeglicher R₅- und R₈-Acylgruppen durch Deacylierung und wahlweise
b) Entfernen von jeglicher Einheit 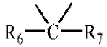 und von jeglicher Einheit 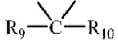 durch Behandlung mit einer Säure.

12. Verfahren nach Anspruch 11, wobei R₁ aus Benzyl, 4-Methylbenzyl, Naphthylmethyl, 4-Phenylbenzyl, 4-Chlorbenzyl, 4-Methoxybenzyl, 3,4-Dimethoxybenzyl, 2,4,6-Trimethylbenzyl und 2,3,4,5,6-Pentamethylbenzyl ausgewählt ist, R₂ aus Benzyl, 4-Methylbenzyl, Naphthylmethyl, Benzyloxycarbonyl, 4-Phenylbenzyl, 4-Chlorbenzyl, 4-Methoxybenzyl, 3,4-Dimethoxybenzyl, 2,4,6-Trimethylbenzyl und 2,3,4,5,6-Pentamethylbenzyl ausgewählt ist, R₃ aus Benzyl, 4-Methylbenzyl, Naphthylmethyl, Benzyloxycarbonyl, 4-Phenylbenzyl, 4-Chlorbenzyl, 4-Methoxybenzyl, 3,4-Dimethoxybenzyl, 2,4,6-Trimethylbenzyl, 2,3,4,5,6-Pentamethylbenzyl und H ausgewählt ist, R₄ aus Acetyl, Benzoyl und 4-Chlorbenzoyl ausgewählt ist, zwei R₅-Gruppen zusammen Isopropyliden or Cyclohexyliden bilden und R₈ aus Benzyl, 4-Methylbenzyl, Naphthylmethyl, Benzyloxycarbonyl, 4-Phenylbenzyl, 4-Chlorbenzyl, 4-Methoxybenzyl, 3,4-Dimethoxybenzyl, 2,4,6-Trimethylbenzyl, 2,3,4,5,6-Pentamethylbenzyl, Acetyl, Pivaloyl, Benzoyl und 4-Chlorbenzoyl ausgewählt ist.

13. Verfahren nach Anspruch 12, wobei R₁ und R₂ unabhängig aus Benzyl und 4-Methylbenzyl ausgewählt sind, R₃ aus Benzyl, 4-Methylbenzyl und H ausgewählt ist und R₈ aus Benzyl, 4-Methylbenzyl, Acetyl, Pivaloyl, Benzoyl und 4-Chlorbenzoyl ausgewählt ist.

14. Verbindung mit der Formel 2A 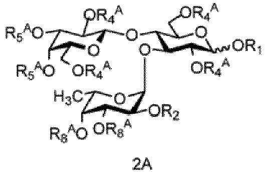 wobei R₁ und R₂ unabhängig für eine Gruppe stehen, die durch Hydrogenolyse entfernbar ist, R₄^{A} aus Acyl und H ausgewählt ist, R₅^{A} aus Acyl und H ausgewählt ist oder zwei R₅^{A}-Gruppen zusammen eine Einheit 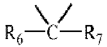 bilden, wobei R₆ und R₇ unabhängig aus Alkyl und Phenyl ausgewählt sind oder wobei R₆ und R₇ zusammen mit dem Kohlenstoffatom, an dem sie gebunden sind, ein Cycloalkyliden bilden, und R₈^{A} aus Acyl und H ausgewählt ist oder zwei R₈^{A}-Gruppen zusammen eine Einheit 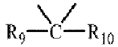 bilden, wobei R₉ und R₁₀ unabhängig aus Alkyl und Phenyl ausgewählt sind oder wobei R₉ und R₁₀ zusammen mit dem Kohlenstoffatom, an dem sie gebunden sind, ein Cycloalkyliden bilden, vorausgesetzt, dass R₄^{A}, R₅^{A} und R₈^{A} nicht alle für H stehen,
oder ein Hydrat oder Solvat davon.

15. Verbindung nach Anspruch 14, wobei R₁ aus Benzyl, 4-Methylbenzyl, Naphthylmethyl, 4-Phenylbenzyl, 4-Chlorbenzyl, 4-Methoxybenzyl, 3,4-Dimethoxybenzyl, 2,4,6-Trimethylbenzyl und 2,3,4,5,6-Pentamethylbenzyl, vorzugsweise aus Benzyl und 4-Methylbenzyl, ausgewählt ist und R₂ aus Benzyl, 4-Methylbenzyl, Naphthylmethyl, Benzyloxycarbonyl, 4-Phenylbenzyl, 4-Chlorbenzyl, 4-Methoxybenzyl, 3,4-Dimethoxybenzyl, 2,4,6-Trimethylbenzyl und 2,3,4,5,6-Pentamethylbenzyl, vorzugsweise aus Benzyl und 4-Methylbenzyl, ausgewählt ist, R₄^{A} aus Acetyl, Pivaloyl, Benzoyl, 4-Chlorbenzoyl und H ausgewählt ist, R₅^{A} für H steht oder zwei R₅^{A}-Gruppen zusammen ein Isopropyliden oder ein Cyclohexyliden bilden, R₈^{A} aus Acetyl, Pivaloyl, Benzoyl, 4-Chlorbenzoyl und H ausgewählt ist und -OR₁ sich in β-Stellung befindet.

## Revendications

1. Composé de formule 1 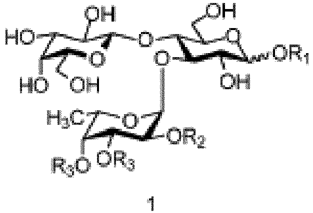 dans lequel R1 et R2 sont indépendamment un groupe éliminable par hydrogénolyse ; et R3 est choisi parmi un groupe éliminable par hydrogénolyse et H ;
ou hydrate ou solvate de celui-ci.

2. Composé selon la revendication 1, dans lequel R1 est choisi parmi le benzyle, le 4-méthylbenzyle, le naphtylméthyle, le 4-phénylbenzyle, le 4-chlorobenzyle, le 4-méthoxybenzyle, le 3,4-diméthoxybenzyle, le 2,4,6-triméthylbenzyle et le 2,3,4,5,6-pentaméthylbenzyle ; R2 est choisi parmi le benzyle, le 4-méthylbenzyle, le naphtylméthyle, le benzyloxycarbonyle, le 4-phénylbenzyle, le 4-chlorobenzyle, le 4-méthoxybenzyle, le 3,4-diméthoxybenzyle, le 2,4,6-triméthylbenzyle et le 2,3,4,5,6-pentaméthylbenzyle ; et R3 est choisi parmi le benzyle, le 4-méthylbenzyle, le naphtylméthyle, le benzyloxycarbonyle, le 4-phénylbenzyle, le 4-chlorobenzyle, le 4-méthoxybenzyle, le 3,4-diméthoxybenzyle, le 2,4,6-triméthylbenzyle, le 2,3,4,5,6-pentaméthylbenzyle et H.

3. Composé selon la revendication 2, dans lequel R1 et R2 sont indépendamment choisis parmi le benzyle et le 4-méthylbenzyle ; R3 est choisi parmi le benzyle, le 4-méthylbenzyle et H ; et -OR1 est dans le sens β.

4. Procédé de production de 3-O-fucosyllactose comprenant l'étape consistant à soumettre à hydrogénolyse un composé de formule 1 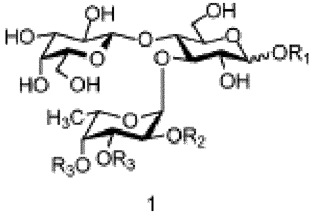 dans lequel R1 et R2 sont indépendamment un groupe éliminable par hydrogénolyse ; et R3 est choisi parmi un groupe éliminable par hydrogénolyse et H ;
ou hydrate ou solvate de celui-ci.

5. Procédé selon la revendication 4, dans lequel l'hydrogénolyse est effectuée dans l'eau, dans un ou plusieurs alcools en C1-C6 ou dans un mélange d'eau et d'un ou de plusieurs alcools en C1-C6, en présence d'un catalyseur à base de palladium ou de nickel de Raney.

6. Procédé selon la revendication 5, dans lequel ledit palladium est du palladium sur charbon ou du noir de palladium.

7. Procédé selon l'une quelconque des revendications 4 à 6, dans lequel, dans le composé de formule 1, R1 est choisi parmi le benzyle, le 4-méthylbenzyle, le naphtylméthyle, le 4-phénylbenzyle, le 4-chlorobenzyle, le 4-méthoxybenzyle, le 3,4-diméthoxybenzyle, le 2,4,6-triméthylbenzyle et le 2,3,4,5,6-pentaméthylbenzyle ; R2 est choisi parmi le benzyle, le 4-méthylbenzyle, le naphtylméthyle, le benzyloxycarbonyle, le 4-phénylbenzyle, le 4-chlorobenzyle, le 4-méthoxybenzyle, le 3,4-diméthoxybenzyle, le 2,4,6-triméthylbenzyle et le 2,3,4,5,6-pentaméthylbenzyle ; et R3 est choisi parmi le benzyle, le 4-méthylbenzyle, le naphtylméthyle, le benzyloxycarbonyle, le 4-phénylbenzyle, le 4-chlorobenzyle, le 4-méthoxybenzyle, le 3,4-diméthoxybenzyle, le 2,4,6-triméthylbenzyle, le 2,3,4,5,6-pentaméthylbenzyle et H.

8. Procédé selon la revendication 7, dans lequel R1 et R2 sont indépendamment choisis parmi le benzyle et le 4-méthylbenzyle ; R3 est choisi parmi le benzyle, le 4-méthylbenzyle et H ; et -OR1 est dans le sens β.

9. Procédé selon l'une quelconque des revendications 4 à 8, comprenant en outre l'étape précédente consistant à :
- convertir un composé de formule 2 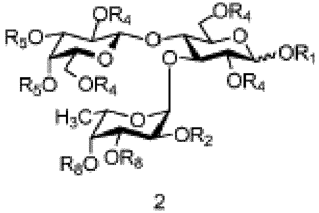 dans lequel R1 et R2 sont tels que définis ci-dessus ; R4 est un groupe acyle ; R5 est un groupe acyle ou deux groupes R5 forment conjointement un fragment 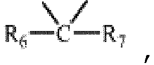 dans lequel R6 et R7 sont indépendamment choisis parmi un alkyle et le phényle, ou dans lequel R6 et R7, conjointement avec l'atome de carbone auquel ils sont liés, forment un cycloalkylidène ; et R8 est choisi parmi un groupe éliminable par hydrogénolyse et un acyle, ou les deux groupes R8 forment conjointement un fragment 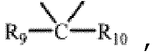 dans lequel R9 et R10 sont indépendamment choisis parmi un alkyle et le phényle, ou dans lequel R9 et R10, conjointement avec l'atome de carbone auquel ils sont liés, forment un cycloalkylidène,
en un composé de formule 1 ou en un hydrate ou un solvate de celui-ci par :
a) désacylation des groupes R4 acyles et de l'un quelconque des groupes R5 et R8 acyles du composé de formule 2 ; et facultativement
b) élimination d'un quelconque fragment 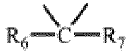 et d'un quelconque fragment 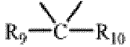 du composé de formule 2 par traitement avec un acide.

10. Procédé selon la revendication 9, dans lequel R4 est choisi parmi l'acétyle, le benzoyle et le 4-chlorobenzoyle, deux groupes R5 forment conjointement un isopropylidène ou un cyclohexylidène et R8 est choisi parmi le benzyle, le 4-méthylbenzyle, le naphtylméthyle, le benzyloxycarbonyle, le 4-phénylbenzyle, le 4-chlorobenzyle, le 4-méthoxybenzyle, le 3,4-diméthoxybenzyle, le 2,4,6-triméthylbenzyle, le 2,3,4,5,6-pentaméthylbenzyle, l'acétyle, le pivaloyle, le benzoyle et le 4-chlorobenzoyle, de préférence le benzyle, le 4-méthylbenzyle, l'acétyle, le pivaloyle, le benzoyle et le 4-chlorobenzoyle.

11. Procédé de production d'un composé de formule 1 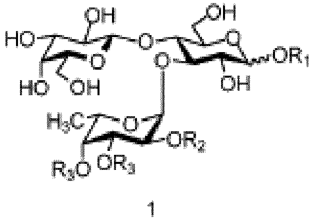 dans lequel R1 et R2 sont indépendamment un groupe éliminable par hydrogénolyse, et R3 est choisi parmi un groupe éliminable par hydrogénolyse et H ; ou hydrate ou solvate de celui-ci, à partir d'un composé de formule 2 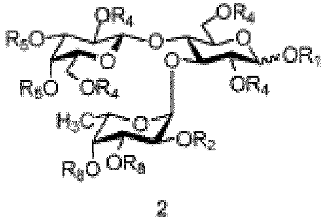 dans lequel R1 et R2 sont tels que définis ci-dessus ; R4 est un groupe acyle ; R5 est un groupe acyle ou deux groupes R5 forment conjointement un fragment 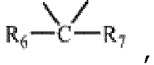 dans lequel R6 et R7 sont indépendamment choisis parmi un alkyle et le phényle, ou dans lequel R6 et R7, conjointement avec l'atome de carbone auquel ils sont liés, forment un cycloalkylidène ; et R8 est choisi parmi un groupe éliminable par hydrogénolyse et l'acyle, ou deux groupes R8 forment conjointement un fragment 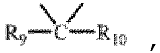 dans lequel R9 et R10 sont indépendamment choisis parmi un alkyle et le phényle, ou dans lequel R9 et R10, conjointement avec l'atome de carbone auquel ils sont liés, forment un cycloalkylidène,
comprenant les étapes consistant à :
a) éliminer, par désacylation, les groupes R4 acyles et l'un quelconque des groupes R5 et R8 acyles ; et facultativement
b) éliminer un quelconque fragment 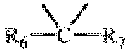 et un quelconque fragment 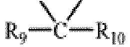 par traitement avec un acide.

12. Procédé selon la revendication 11, dans lequel R1 est choisi parmi le benzyle, le 4-méthylbenzyle, le naphtylméthyle, le 4-phénylbenzyle, le 4-chlorobenzyle, le 4-méthoxybenzyle, le 3,4-diméthoxybenzyle, le 2,4,6-triméthylbenzyle et le 2,3,4,5,6-pentaméthylbenzyle ; R2 est choisi parmi le benzyle, le 4-méthylbenzyle, le naphtylméthyle, le benzyloxycarbonyle, le 4-phénylbenzyle, le 4-chlorobenzyle, le 4-méthoxybenzyle, le 3,4-diméthoxybenzyle, le 2,4,6-triméthylbenzyle et le 2,3,4,5,6-pentaméthylbenzyle ; R3 est choisi parmi le benzyle, le 4-méthylbenzyle, le naphtylméthyle, le benzyloxycarbonyle, le 4-phénylbenzyle, le 4-chlorobenzyle, le 4-méthoxybenzyle, le 3,4-diméthoxybenzyle, le 2,4,6-triméthylbenzyle, le 2,3,4,5,6-pentaméthylbenzyle et H ; R4 est choisi parmi l'acétyle, le benzoyle et le 4-chlorobenzoyle ; deux groupes R5 forment conjointement un isopropylidène ou un cyclohexylidène et R8 est choisi parmi le benzyle, le 4-méthylbenzyle, le naphtylméthyle, le benzyloxycarbonyle, le 4-phénylbenzyle, le 4-chlorobenzyle, le 4-méthoxybenzyle, le 3,4-diméthoxybenzyle, le 2,4,6-triméthylbenzyle, le 2,3,4,5,6-pentaméthylbenzyle, l'acétyle, le pivaloyle, le benzoyle et le 4-chlorobenzoyle.

13. Procédé selon la revendication 12, dans lequel R1 et R2 sont indépendamment choisis parmi le benzyle et le 4-méthylbenzyle ; R3 est choisi parmi le benzyle, le 4-méthylbenzyle et H et R8 est choisi parmi le benzyle, le 4-méthylbenzyle, l'acétyle, le pivaloyle, le benzoyle et le 4-chlorobenzoyle.

14. Composé de formule 2A 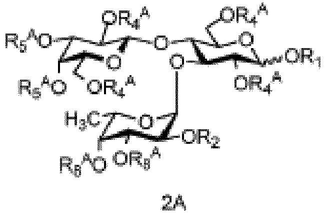 dans lequel R1 et R2 sont indépendamment un groupe éliminable par hydrogénolyse ; R4A est choisi parmi un acyle et H ; R5A est choisi parmi un acyle et H, ou deux groupes R5A forment conjointement un fragment 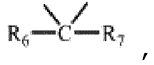 dans lequel R6 et R7 sont indépendamment choisis parmi un alkyle et le phényle, ou dans lequel R6 et R7, conjointement avec l'atome de carbone auquel ils sont liés, forment un cycloalkylidène ; et R8A est choisi parmi un acyle et H, ou deux groupes R8A forment conjointement un fragment 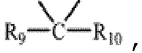 dans lequel R9 et R10 sont indépendamment choisis parmi un alkyle et le phényle, ou dans lequel R9 et R10, conjointement avec l'atome de carbone auquel ils sont liés, forment un cycloalkylidène, à condition que R4A, R5A et R8A ne soient pas tous H ;
ou hydrate ou solvate de celui-ci.

15. Composé selon la revendication 14, dans lequel R1 est choisi parmi le benzyle, le 4-méthylbenzyle, le naphtylméthyle, le 4-phénylbenzyle, le 4-chlorobenzyle, le 4-méthoxybenzyle, le 3,4-diméthoxybenzyle, le 2,4,6-triméthylbenzyle et le 2,3,4,5,6-pentaméthylbenzyle, de préférence le benzyle et le 4-méthylbenzyle ; et R2 est choisi parmi le benzyle, le 4-méthylbenzyle, le naphtylméthyle, le benzyloxycarbonyle, le 4-phénylbenzyle, le 4-chlorobenzyle, le 4-méthoxybenzyle, le 3,4-diméthoxybenzyle, le 2,4,6-triméthylbenzyle et le 2,3,4,5,6-pentaméthylbenzyle, de préférence le benzyle et le 4-méthylbenzyle ; R4A est choisi parmi l'acétyle, le pivaloyle, le benzoyle, le 4-chlorobenzoyle et H ; R5A est H, ou deux groupes R5A forment conjointement un isopropylidène ou un cyclohexylidène ; R8A est choisi parmi l'acétyle, le pivaloyle, le benzoyle, le 4-chlorobenzoyle et H ; et -OR1 est dans le sens β.
